# EUROPEAN PATENT APPLICATION

(11) **EP 4 592 674 A1**
(43) Date of publication of application: **30.07.2025**
(21) Application number: 24875661.1
(22) Date of filing: 26.11.2024
(51) Int. Cl.: G01N 33/18, G01N 1/10, G01N 1/40

(54) **ANALYSIS DEVICE AND ANALYSIS METHOD**

(30) Priority: 13.12.2023 JP 2023210477
(71) Applicant: Nomura Micro Science Co., Ltd., Atsugi-shi, Kanagawa 243-0021 (JP); Sophia School Corporation, Tokyo 102-8554 (JP)
(72) Inventor: KIMOTO, Hiroshi, Atsugi-Shi, Kanagawa 2430021 (JP); IIYAMA, Masamitsu, Atsugi-Shi, Kanagawa 2430021 (JP); HASHIMOTO, Takeshi, Tokyo 1028554 (JP); HAYASHITA, Takashi, Tokyo 1028554 (JP)
(74) Representative: DREISS Patentanwälte PartG mbB
(86) International application number: PCT/JP2024/041887
(87) International publication number: WO 2025/126836

(57) **Abstract**

An analysis device includes: a membrane filtration device provided with a microfiltration membrane or an ultrafiltration membrane; a flow injection system that analyzes sample water from a reaction between a detection target that is included in the sample water, and a reagent that is included in carrier water and that reacts with the detection target; first piping that passes concentrated water, in which the detection target has been concentrated by the membrane filtration device, to the flow injection system as the sample water; and a sample water pump that pressurizes the sample water in the first piping, an internal pipe diameter of an outlet pipe in the flow injection system being smaller than an internal pipe diameter of the first piping.

## Description

### Technical Field

The present disclosure relates to an analysis device and an analysis method.

### Background Art

Japanese Patent Application Laid-Open (JP-A) No. S62-280652 describes a flow injection analysis (hereafter abbreviated to "FIA") method for analyzing an element to be detected by guiding a liquid-liquid mixture configured from an organic phase and a water phase either to inside an inner pipe of double-walled piping configured from multi-pore polymer membrane inner pipe and an outer pipe encompassing the inner tubing, or to a region between the inner pipe and the outer pipe, passing only the organic phase through a wall face of the multi-pore polymer membrane inner pipe so as to separate the organic phase from the water phase, and introducing the organic phase obtained thereby into a detection section to analyze the element to be detected.

The inventors performed low concentration analysis of endotoxins and the like using an FIA method that employs a florescent substance such as described in JP-A No. 2022-011525. However, measurement to as far as the low concentrations found when measuring online was difficult.

### SUMMARY OF INVENTION

### Technical Problem

Generally speaking, endotoxins are substances that should not be contained as pyrogens in water employed in drug manufacture, such as purified water, water for injection (WFI), and the like. This means that not only do they need to be removed as much as possible when producing purified water, water for injection (WFI), and the like, but there is also a need to monitor for the presence of endotoxins online. In online measurements, as well as being able to quantify down to low concentrations, there is also demand to be able to measure accurately and stably.

In an online analysis device and analysis method using an FIA method, when performing an analysis of products derived from bacteria including endotoxins, performing analysis quantitatively is difficult when the concentration of products derived from bacteria is low.

An object of the present disclosure is to raise the ability to perform quantitative analysis in an online analysis device and analysis method using an FIA method.

### Solution to Problem

An analysis device of a first aspect includes: a membrane filtration device provided with a microfiltration membrane or an ultrafiltration membrane; a flow injection system that analyzes sample water from a reaction between a detection target contained in the sample water and a reagent that is contained in carrier water and that reacts with the detection target; first piping that passes concentrated water, in which the detection target has been concentrated by the membrane filtration device, to the flow injection system as the sample water; and a sample water pump that pressurizes the sample water in the first piping, wherein an internal pipe diameter of an outlet pipe in the flow injection system is smaller than an internal pipe diameter of the first piping.

In this analysis device, the membrane filtration device includes either the microfiltration membrane or the ultrafiltration membrane. The concentrated water, in which the detection target has been concentrated, is obtained by passing analysis target water through the membrane filtration device. The concentrated water is pressurized by the sample water pump on the first piping and passed to the flow injection system as the sample water. In the flow injection system, the sample water is analyzed from the reaction with the reagent contained in the carrier water. The concentrated water, in which the detection target has been concentrated by the membrane filtration device, is accordingly fed to the flow injection system as the sample water, enabling a raised ability to perform quantitative analysis of the sample water on a detection target of products derived from bacteria including endotoxins.

Furthermore, in this analysis device, the internal pipe diameter of the outlet pipe in the flow injection system is smaller than the internal pipe diameter of the first piping.

The water pressure of the sample water at the downstream side of the pump on the first piping can accordingly be maintained in a higher pressure state than the water pressure at the upstream side of the pump. The flow rate of the sample water ejected from the pump can be stabilized, and so the concentration factor of the detection target of sample water can accordingly be raised.

An analysis device of a second aspect includes: a membrane filtration device provided with a microfiltration membrane or an ultrafiltration membrane; a flow injection system that analyzes sample water from a reaction between a detection target contained in the sample water and a reagent that is contained in carrier water and that reacts with the detection target; first piping that passes concentrated water, in which the detection target has been concentrated by the membrane filtration device, to the flow injection system as the sample water; second piping that passes at least part of pass-through water of the membrane filtration device to the flow injection system as the carrier water; and a sample water pump that pressurizes the sample water in the first piping.

**In** this analysis device, the membrane filtration device includes either the microfiltration membrane or the ultrafiltration membrane. The concentrated water, in which the detection target has been concentrated, is obtained by passing the analysis target water through the membrane filtration device. The concentrated water is pressurized by the sample water pump on the first piping and passed to the flow injection system as sample water. **In** the flow injection system, the sample water is analyzed from a reaction with the reagent contained in the carrier water. The concentrated water, in which the detection target has been concentrated by the membrane filtration device, is accordingly fed to the flow injection system as the sample water, and this accordingly enables a raised ability to perform quantitative analysis of the sample water on a detection target of products derived from bacteria including endotoxins.

Furthermore, this analysis device also includes the second piping that passes at least part of the pass-through water of the membrane filtration device to the flow injection system as the carrier water.

This means that there is no need for a device to obtain the carrier water, enabling a simplification in the configuration of the analysis device to be achieved.

An analysis device of a third aspect includes: a membrane filtration device provided with a microfiltration membrane or an ultrafiltration membrane; a flow injection system that analyzes sample water from a reaction between a detection target contained in the sample water and a reagent that is contained in carrier water and that reacts with the detection target; first piping that passes concentrated water, in which the detection target has been concentrated by the membrane filtration device, to the flow injection system as the sample water; second piping that passes the carrier water to the flow injection system; and a sample water pump that pressurizes the sample water in the first piping, wherein the membrane filtration device includes a connection port to which the second piping is connected.

**In** this analysis device, the membrane filtration device includes either the microfiltration membrane or the ultrafiltration membrane. The concentrated water, in which the detection target has been concentrated, is obtained by passing the analysis target water through the membrane filtration device. The concentrated water is pressurized by the sample water pump on the first piping and passed to the flow injection system as the sample water. **In** the flow injection system, the sample water is analyzed from a reaction with the reagent contained in the carrier water. **In** this manner, the concentrated water, in which the detection target has been concentrated by the membrane filtration device, is fed to the flow injection system as the sample water, enabling a raised ability to perform quantitative analysis of the sample water on a detection target of products derived from bacteria including endotoxins.

Furthermore, this analysis device also includes the second piping that passes at least part of the pass-through water of the membrane filtration device to the flow injection system as the carrier water.

This means that a device to obtain the carrier water is not needed, enabling a simplification in the configuration of the analysis device to be achieved.

In this analysis device, the membrane filtration device also includes the connection port to which the second piping is connected.

By connecting the second piping to the connection port of the membrane filtration device, the pass-through water of the membrane filtration device can be fed to the second piping as the carrier water without being exposed to external air, enabling foreign matter to be suppressed from being incorporated in the carrier water.

An analysis device of the fourth aspect includes: a membrane filtration device provided with a microfiltration membrane or an ultrafiltration membrane; a flow injection system that analyzes sample water from a reaction between a detection target contained in the sample water and a reagent that is contained in carrier water and that reacts with the detection target; first piping that passes concentrated water, in which the detection target has been concentrated by the membrane filtration device, to the flow injection system as the sample water; and a sample water pump that pressurizes the sample water in the first piping, wherein a pressure at an inlet side of the membrane filtration device is less than a pressure at an outlet port of the sample water pump and is 0.05 MPa or higher.

In this analysis device, the membrane filtration device includes either the microfiltration membrane or the ultrafiltration membrane. The concentrated water, in which the detection target has been concentrated, is obtained by passing the analysis target water through the membrane filtration device. The concentrated water is pressurized by the sample water pump on the first piping and passed to the flow injection system as the sample water. In the flow injection system, the sample water is analyzed from a reaction with the reagent contained in the carrier water. In this manner, the concentrated water, in which the detection target has been concentrated by the membrane filtration device, is fed to the flow injection system as the sample water, enabling a raised ability to perform quantitative analysis of the sample water on a detection target of products derived from bacteria including endotoxins.

Furthermore, in this analysis device, the pressure at the inlet side of the membrane filtration device is less than the pressure at the outlet port of the sample water pump and is 0.05 MPa or higher.

Setting the pressure at the inlet side of the membrane filtration device in this manner enables a raised ability to perform quantitative analysis of the sample water.

An analysis device of a fifth aspect is the second or third aspect, wherein the membrane filtration device includes a discharge section that is open externally and that discharges pass-through water that has passed through the microfiltration membrane or the ultrafiltration membrane, and a linear velocity of the pass-through water discharged from the discharge section is from 0.1 m/s to 2.0 m/s.

Due to a large part of the pass-through water that has passed through the ultrafiltration membrane of the membrane filtration device being discharged from the discharge section, the rate of discharge of the pass-through water per unit time in the membrane filtration device is increased, enabling the concentration of the concentrated water to be raised.

Moreover, by setting the linear velocity of the pass-through water in this manner, a sufficient volume of pass-through water can be obtained, enabling endotoxins to be sufficiently concentrated in the membrane filtration device.

An analysis device of a sixth aspect is the first or the fourth aspect, wherein the membrane filtration device includes a discharge section that is open externally and that discharges pass-through water that has passed through the microfiltration membrane or the ultrafiltration membrane, a linear velocity of the pass-through water discharged from the discharge section is from 0.1 m/s to 2.0 m/s, and the analysis device further includes second piping that passes at least part of the pass-through water to the flow injection system as the carrier water.

Due to a large part of the pass-through water passed through the ultrafiltration membrane of the membrane filtration device being discharged from the discharge section, the rate of discharge of the pass-through water per unit time in the membrane filtration device is increased, enabling the concentration of the concentrated water to be raised.

Moreover, due to setting the linear velocity of the pass-through water in this manner, a sufficient volume of pass-through water can be obtained, enabling endotoxins to be sufficiently concentrated in the membrane filtration device.

Due to providing the second piping, a device to obtain the carrier water is not needed, enabling a simplification in the configuration of the analysis device to be achieved.

An analysis method of a seventh aspect includes generating sample water in which a detection target has been concentrated by a membrane filtration device provided with a microfiltration membrane or an ultrafiltration membrane, pressurizing the sample water with a sample water pump and feeding the sample water to a flow injection system, analyzing the sample water in the flow injection system from a reaction between the detection target and a reagent that is contained in carrier water and that reacts with the detection target, and setting a water pressure at a downstream side of the sample water pump higher than a water pressure at an upstream side of the sample water pump.

**In** this analysis method, the membrane filtration device includes either the microfiltration membrane or the ultrafiltration membrane. The concentrated water, in which the detection target has been concentrated, is obtained by passing the analysis target water through the membrane filtration device. The concentrated water is pressurized by the sample water pump and fed to the flow injection system as sample water. **In** the flow injection system, the detection target is analyzed from the reaction with the reagent contained in the carrier water. **In** this manner, the concentrated water, in which the detection target has been concentrated by the membrane filtration device, is fed to the flow injection system as the sample water, thereby enabling an ability to perform quantitative analysis on a detection target of products derived from bacteria including endotoxins be raised.

Moreover, in this analysis method, the water pressure at the downstream side of the pump is higher than the water pressure at the upstream side of the pump.

This thereby enables the flow rate of the sample water ejected from the pump to be stabilized, enabling the concentration factor of the detection target in the sample water to be raised.

An analysis method of an eighth aspect includes generating sample water in which a detection target has been concentrated by a membrane filtration device provided with a microfiltration membrane or an ultrafiltration membrane, pressurizing the sample water with a sample water pump and feeding the sample water to a flow injection system, feeding at least part of pass-through water that has passed through the microfiltration membrane or the ultrafiltration membrane in the membrane filtration device as carrier water to the flow injection system, and analyzing the sample water in the flow injection system from a reaction between the detection target and a reagent that is contained in the carrier water and that reacts with the detection target.

In this analysis method, the membrane filtration device includes either the microfiltration membrane or the ultrafiltration membrane. Sample water is obtained from the concentrated water, in which the detection target has been concentrated, by passing the analysis target water through the membrane filtration device. The sample water is pressurized by the sample water pump and fed to the flow injection system. At least part of the pass-through water that has passed through the microfiltration membrane or the ultrafiltration membrane in the membrane filtration device is fed to the flow injection system as the carrier water. In the flow injection system, the detection target of the sample water is analyzed from the reaction with the reagent contained in the carrier water. The concentrated water, in which the detection target has been concentrated by the membrane filtration device, is fed to the flow injection system as the sample water, thereby enabling an ability to perform quantitative analysis on a detection target of products derived from bacteria including endotoxins to be raised.

Moreover, at least part of the pass-through water that has passed through the microfiltration membrane or the ultrafiltration membrane in the membrane filtration device is fed to the flow injection system as the carrier water, and so a device to obtain the carrier water is not needed, enabling a simplification in the configuration of the analysis device to be achieved.

An analysis method of a ninth aspect includes generating sample water in which a detection target has been concentrated by a membrane filtration device provided with a microfiltration membrane or an ultrafiltration membrane, pressurizing the sample water with a sample water pump and feeding the sample water to a flow injection system, setting a pressure at an inlet side of the membrane filtration device to less than a pressure at an outlet port of the sample water pump and to 0.05 MPa or higher, and analyzing the sample water in the flow injection system from a reaction between the detection target and a reagent that is contained in carrier water and that reacts with the detection target.

In this analysis method, the membrane filtration device includes either the microfiltration membrane or the ultrafiltration membrane. Sample water is obtained from the concentrated water, in which the detection target has been concentrated, by passing the analysis target water through the membrane filtration device. The sample water is pressurized by the sample water pump and fed to the flow injection system. In the flow injection system, the detection target of the sample water is analyzed from the reaction with the reagent contained in the carrier water. The concentrated water, in which the detection target has been concentrated by the membrane filtration device, is fed to the flow injection system as the sample water, thereby enabling an ability to perform quantitative analysis on a detection target of products derived from bacteria including endotoxins to be raised.

Moreover, setting the pressure at the inlet side of the membrane filtration device to less than the pressure at the outlet port of the sample water pump and to 0.05 MPa or higher enables an ability to perform quantitative analysis of the sample water to be raised.

An analysis method of a tenth aspect is the eighth aspect, wherein in the membrane filtration device, pass-through water that has passed through the microfiltration membrane or the ultrafiltration membrane is externally discharged in an unpressurized state from a discharge section of the membrane filtration device and at a linear velocity of from 0.1 m/s to 2.0 m/s.

A sufficient volume of pass-through water can be obtained by setting the linear velocity of the pass-through water in this manner, enabling endotoxins to be sufficiently concentrated in the membrane filtration device.

An analysis method of an eleventh aspect is the seventh aspect or the ninth aspect, wherein, in the membrane filtration device, pass-through water that has passed through the microfiltration membrane or the ultrafiltration membrane is externally discharged in an unpressurized state from a discharge section of the membrane filtration device and at a linear velocity of from 0.1 m/s to 2.0 m/s, and at least part of the pass-through water that has passed through the microfiltration membrane or the ultrafiltration membrane of the membrane filtration device is fed as carrier water to the flow injection system.

By setting the linear velocity of the pass-through water in this manner, a sufficient volume of pass-through water can be obtained, enabling endotoxins to be sufficiently concentrated in the membrane filtration device.

Moreover, at least part of the pass-through water that has passed through the microfiltration membrane or the ultrafiltration membrane of the membrane filtration device is fed as carrier water to the flow injection system, and so a device to obtain the carrier water is not needed, enabling a simplification in the configuration of the analysis device to be achieved.

### Advantageous Effects

The technology disclosed herein enables an ability to perform quantitative analysis to be raised in an analysis device and an analysis method using an FIA method.

### BRIEF DESCRIPTION OF DRAWINGS

Fig. 1 is a configuration diagram illustrating an analysis device of a first exemplary embodiment.
Fig. 2 is a configuration diagram illustrating an analysis device of a modified example of the first exemplary embodiment.
Fig. 3 is a configuration diagram illustrating an analysis device of an Example 8.

### DESCRIPTION OF EMBODIMENTS

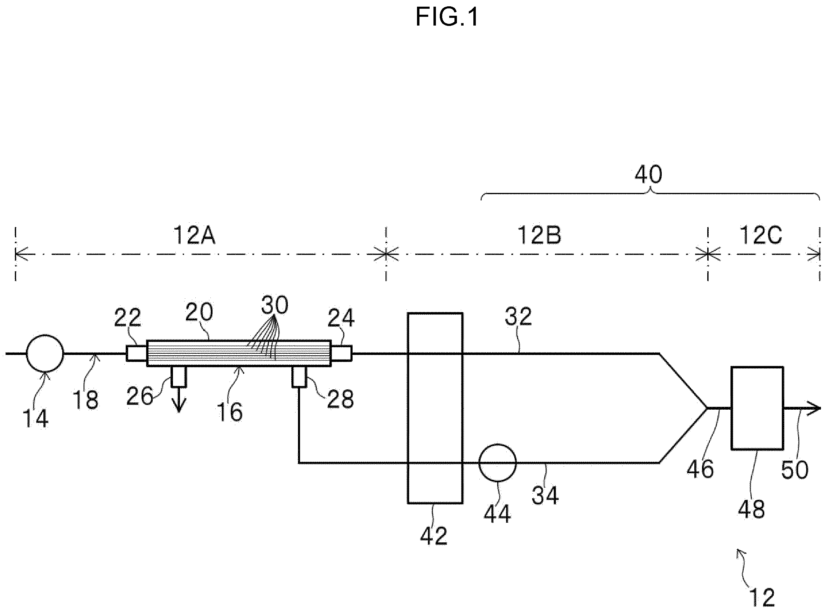

Description follows regarding an analysis device 12 according to a first exemplary embodiment, with reference to the drawings. As illustrated in Fig. 1, in the analysis device 12, water obtained in a non-illustrated water treatment system, or water being treated, is employed as the target for analysis. In the present exemplary embodiment, the analysis device 12 analyzes endotoxins contained in the analysis target. Endotoxins are an example of a product derived from bacteria, and an example of a detection target of technology disclosed herein. Namely, in the analysis device 12 and the analysis method of technology disclosed herein, a product derived from bacteria, including endotoxins, is the detection target. The detection target is not limited to endotoxins, namely to lipopolysaccharides as applied to configure cell walls of gram-negative bacteria and may, for example, be various types of bacteria (the bacteria themselves).

Note that examples of water obtained by a water treatment system include, for example, water for injection, water for drug manufacture, and the like, however there is no limitation thereto. Examples of the water treatment system include, specifically, for example, a drug manufacture water production facility or the like for purified water, water for injection (WFI), or the like.

The analysis device 12 is configured including a front concentration section 12A, a reagent mixing section 12B, and a detection section 12C.

The front concentration section 12A includes a front concentration pump 14 and a membrane filtration device 16 in the example illustrated. Analysis target water is fed from the water treatment system to the front concentration pump 14. The front concentration pump 14 pressurizes the analysis target water, and feeds the pressurized target water through piping 18 to the membrane filtration device 16 at a later stage. Note that, for example, the front concentration pump 14 is not needed in cases in which the analysis device 12 is directly attached to a sampling line of a purified water manufacturing device or an ultrapure water manufacturing device for online measurement, as long as the pressure of the sampling line is appropriate. Moreover, a pressure reduction means, such as a pressure reduction valve or the like, may be installed instead of the front concentration pump 14 in cases in which the pressure of the sampling line is too much higher than an appropriate pressure.

The membrane filtration device 16 includes an outer pipe 20. An inlet port 22 is provided at one length direction end of the outer pipe 20, and an outlet port 24 is provided at the other end thereof. A discharge section 26 and a water feed section 28 are also provided at an outer periphery of the outer pipe 20. The piping 18 from the front concentration pump 14 is connected to the inlet port 22. First piping 32, described later, is connected to the outlet port 24.

An ultrafiltration membrane 30 including plural hollow fibers is disposed inside the outer pipe 20. Sample water that has inflowed inside the outer pipe 20 from the inlet port 22 is filtered by the ultrafiltration membrane 30. Specifically, since endotoxins do not pass through the ultrafiltration membrane 30, concentrated water, in which the endotoxins have been concentrated, is generated and reaches the outlet port 24. The pore size of the ultrafiltration membrane 30 is from about 0.001 to about 0.01 µm. When products derived from bacteria, including endotoxins, are the detection target as in the technology disclosed herein, the ultrafiltration membrane 30 employed preferably has a pore size set in relation to the size of the product derived from bacteria so as to obtain concentrated water in which the detection target has been concentrated. Note that a microfiltration membrane may be employed instead of the ultrafiltration membrane. As the ultrafiltration membrane or the microfiltration membrane, a membrane having a detection target substance removal rate of 90% or greater is more preferable, and having a removal rate of 95% or greater is even more preferable.

In the present exemplary embodiment, materials that may be employed as the ultrafiltration membrane 30 include cellulose acetate, polyacrylonitrile, polysulfone, poly ether sulfone, modified poly ether sulfone, polyvinylidene fluoride, or the like. In particular, modified poly ether sulfones are not liable to absorb endotoxins, and so are preferable as the material of the ultrafiltration membrane 30.

The membrane filtration device to be employed is not limited and, for example, a KROSFLOW 20CM 5K MPES produced by Repligen may be utilized therefore.

Endotoxins are either not contained, or are contained at extremely minute amounts, in the pass-through water that has passed through the ultrafiltration membrane 30. Namely, this pass-through water is endotoxin-free water. The endotoxin-free water from the discharge section 26 and the water feed section 28 is discharged externally from the membrane filtration device 16. The sample water is concentrated at from 10 times to 100,000 times by the membrane filtration device 16. The concentration factor is more preferably from 1000 times to 100,000 times from the perspective of measurement accuracy.

The discharge port of the discharge section 26 is open. The pass-through water is discharged from the discharge section 26 in a practically unpressurized state. This unpressurized state is one in which atmospheric pressure acts on the pass-through water discharged from the discharge section 26, but pressure exceeding atmospheric pressure does not act thereon.

One end of the first piping 32 is connected to the outlet port 24 of the membrane filtration device 16. Concentrated water, in which endotoxins have been concentrated in the membrane filtration device 16, flows as the sample water in the first piping 32.

In the present exemplary embodiment, the water feed section 24 and a pump 42 are directly attached. Namely, due to there being no reservoir provided between the water feed section 24 and the pump 42, there is no concern regarding contamination of the sample water, or evaporation of the sample water, occurring in a reservoir. Countermeasures, such as increasing the flow rate up to the reservoir, discharging part of the sample water of the reservoir, and the like, are needed to lessen the impact of contamination and evaporation in a reservoir, however in such cases this leads to a concern that there might be a drop in measurement accuracy due a fall in the concentration factor. There is no such concern in the present exemplary embodiment, enabling accurate measurement to be performed.

One end of second piping 34 is connected to the water feed section 28 of the membrane filtration device 16. Endotoxin-free water from which endotoxins have been removed in the membrane filtration device 16 flows as carrier water in the second piping 34. The water feed section 28 is an example of a connection port to which the second piping 34 is connected.

A flow injection system 40 includes the pump 42. In the present exemplary embodiment, the pump 42 is a plunger pump. In this plunger pump, a fluid is pressurized and fed from an upstream side to a downstream side by reciprocal motion of a plunger inside a pump casing. In particular, in the example illustrated in Fig. 1, a double plunger pump including two plungers disposed alongside each other is used as the pump 42 and is provided common to the first piping 32 and the second piping 34. The pump 42 is accordingly able to pressurize and feed both the sample water and the carrier water to the downstream side at the same time. Note that a peristaltic pump, a syringe pump, and a solenoid pump may be employed as the pump 42. A plunger pump is more preferable.

A reagent injection section 44 is provided to the second piping 34 at the downstream side of the pump 42. A reagent that reacts with endotoxins is injected into the carrier water in the reagent injection section 44, and carrier water is obtained thereby.

The first piping 32 and the second piping 34 in the reagent mixing section 12B extend as far as to inside the flow injection system 40. The first piping 32 and the second piping 34 merge together in the interior of the flow injection system 40 to form merge piping 46. The merge piping 46 is included in the detection section 12C.

A florescent reagent that has a florescent site and a recognition site coupled together by a spacer, as described in JP-A No. 2022-011525, may be employed as the reagent of the present application, however there is no particular limitation thereto.

In the merge piping 46, the sample water that flowed in the first piping 32 and the carrier water that flowed in the second piping 34 are merged to configure merged water. In the merged water, a reaction occurs between the endotoxins contained in the sample water and the reagent contained in the carrier water.

An analyzer 48 is installed to the merge piping 46. The analyzer 48 analyzes the concentration of endotoxins from the reaction between the endotoxins and the reagent. In the present exemplary embodiment, non-illustrated piping, and a quartz cell connected to the piping, are disposed in the interior of the analyzer 48. The analyzer 48 shines light onto the merged water flowing in the quartz cell, and detects a concentration of the endotoxins from the intensity of signal of florescent light obtained therefrom.

The analyzer 48 includes an outlet pipe 50 to discharge the merged water after analysis. The inner diameter of the outlet pipe 50 is smaller than the inner diameter of the first piping 32. This means that a state is realized in which a pressure P2 at the downstream side of the pump 42, for example at an outlet port, is higher than a pressure P1 at the inlet side of the pump 42, for example at the upstream side of the membrane filtration device 16.

Quantitative analysis is performed in the analyzer 48 by, for example, shining excitation light having a wavelength of from 340 nm to 360 nm, and measuring florescent light generated at from 440 nm to 520 nm. In the analyzer 48, the flow rate of sample water is from 0.01 to 4.00 mL/minute, with good measurement accuracy achieved by adopting a flow rate in this range.

The materials of the first piping 32, the second piping 34, and the merge piping 46 are not particularly limited as long as they enable the sample water, carrier water, and merged water to respectively flow therein. Similarly, there is no particular limitation to the piping of the analyzer 48 and, for example, by employing a fused silica chip, a reaction can be suppressed between the merged water flowing inside and the piping employed in such a fused silica chip, enabling more accurate analysis.

In the present exemplary embodiment, analysis of the endotoxin concentration is performed by the analyzer 48 while the analysis target water is flowing continuously, realizing a so-called online analysis device and online analysis method.

Next, description follows regarding an operation of the analysis device 12 and an analysis method of the present exemplary embodiment.

The analysis target water fed in from a non-illustrated water treatment system is pressurized by the front concentration pump 14 and fed to the membrane filtration device 16. In the membrane filtration device 16, the analysis target water is filtered by the ultrafiltration membrane 30 to obtain concentrated water in which endotoxins have been concentrated, and pass-through water (endotoxin-free water) not containing endotoxins.

The pass-through water is discharged externally from the membrane filtration device 16 through the discharge section 26 and the outlet port 24. In the analysis device 12 of the present exemplary embodiment, a discharge port of the discharge section 26 is open, and the pass-through water is discharged in an unpressurized state from the discharge section 26. Namely, at least part of the pass-through water that has passed through the ultrafiltration membrane 30 is discharged from the discharge section 26. This means that a greater dischargeable flow rate is achieved than a configuration in which a pressure exceeding atmospheric pressure acts on the pass-through water discharged from the discharge section 26. This enables endotoxins to be concentrated from more treatment target water per unit time, enabling a higher concentration of concentrated water.

Preferably the inner diameter of the discharge section 26 is set to from 2 mm to 30 mm, or the line speed of the pass-through water flowing in the discharge section 26 is set to from 0.1 m/s to 2.0 m/s. Moreover, a length of piping of the discharge section 26 (length from the outer pipe 20 to the discharge port of the discharge section 26) is preferably set to 30 m or less. Setting the shape of the discharge section 26, or the line speed of the pass-through water, in this manner enables pass-through water of sufficient volume to be obtained, enabling endotoxins to be sufficiently concentrated in the membrane filtration device 16. Moreover, an intake pressure of the pump 42 at the downstream side of the water feed section 28 can be maintained in an appropriate range, and the amount of reagent added in the reagent injection section 44 can be maintained at an appropriate amount, further stabilizing measurement values in the analyzer 48. Note that the inner diameter of the discharge section 26 indicates an inner diameter of the discharge section 26 at a portion where the flow path cross-sectional area is at its narrowest, and the line flow speed referred to above is the line speed at this portion.

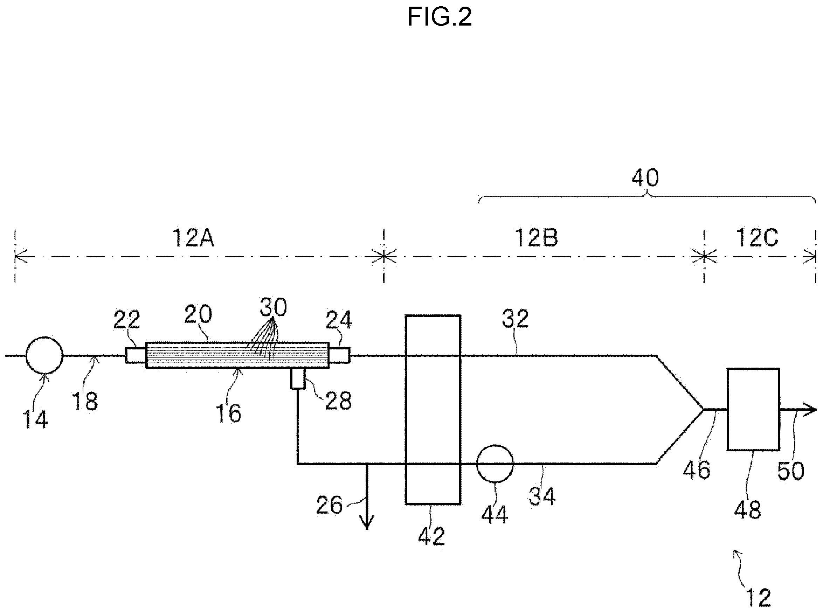

Moreover, as in a first modified example as illustrated in Fig. 2, the discharge section 26 may be provided with a shape that branches from between the water feed section 28 and the pump 42. In such cases, preferably a piping diameter from the water feed section 28 to the discharge section 26 is set in the above range, and the inner diameter of the second piping 34 from the discharge section 26 to the pump 42 is, for example, from 0.5 mm to 2.0 mm.

At least part of the pass-through water passed through the ultrafiltration membrane 30 is fed by the second piping 34 as carrier water from the water feed section 28 to the reagent injection section 44 of the flow injection system 40. There is no need to employ another device to obtain the carrier water, enabling simplification to be achieved in both the configuration of the analysis device 12 and in the analysis method procedure.

Either of the methods of Fig. 1 or Fig. 2 may be employed in cases in which the membrane filtration device 16 is an internal pressure type device, however the method of Fig. 2 is preferably applied for an external pressure type device.

Moreover, the second piping 34 is connected to the discharge section 26, and the pass-through water is not exposed to external air. This thereby enables foreign matter to be suppressed from being incorporated into the pass-through water (carrier water) as the endotoxin-free water.

The carrier water is pressurized by the pump 42 and fed to the downstream side. The reagent is injected by the reagent injection section 44 at the downstream side of the pump 42.

The concentrated water obtained in the membrane filtration device 16 is fed by the first piping 32 as sample water from the outlet port 24 to the reagent injection section 44 of the flow injection system 40. The concentrated water is then pressurized by the pump 42 and fed to the downstream side. In the analysis device 12 of the present exemplary embodiment, concentrated water in which endotoxins have been concentrated to a high concentration by the membrane filtration device 16 is employed as the sample water in this manner, enabling the concentration of the endotoxins to be analyzed by the flow injection system 40. This thereby enables an ability to perform quantitative analysis of endotoxins to be raised compared to cases in which the membrane filtration device 16 is not employed, and sample water having a low concentration of endotoxins is employed.

The first piping 32 is connected to the outlet port 24, and so the concentrated water is not exposed to external air. This thereby enables foreign matter to be suppressed from being incorporated into the concentrated water (sample water).

The sample water of the first piping 32 and the carrier water of the second piping 34 are merged into merged water by the merge piping 46. A reaction then occurs between the endotoxins contained in the sample water and the reagent contained in the carrier water. The merged water is then fed onward to the analyzer 48. The sample water is analyzed in the analyzer 48 to obtain a concentration of the endotoxins from a reaction between endotoxins and the reagent.

In the analysis device 12 of the present exemplary embodiment, the inner diameter of the outlet pipe 50 of the analyzer 48 is set smaller than the inner diameter of the first piping 32. This means that a state is realized in which a pressure P2 of the outlet port at the downstream side of the pump 42 is higher than the pressure P1 at the upstream side of the membrane filtration device 16, which is at the upstream side of the pump 42. An ejection pressure at an outlet side of the pump 42 is not liable to be affected by the water pressure at the inlet side when the pump 42 is driven due to being higher than water pressure at the inlet side of the pump 42. In other words, with respect to an effect on the flow rate of sample water ejected from the pump 42, this is mainly determined by the water pressure at the outlet side. The flow rate of sample water ejected from the pump 42 is no longer affected by fluctuations in pressure at the inlet side, thereby enabling the flow rate at the outlet side to be stabilized and maintained in a fixed range. This means that the concentration factor of the endotoxins in the sample water is higher than cases in which the ejection pressure at the outlet side of the pump 42 is lower than the water pressure at the inlet side of the pump 42. The intensity of signal in the analyzer 48 is also raised as the concentration factor of the endotoxins is raised. Namely, the ability to perform quantitative analysis of endotoxins can be raised in the flow injection system 40.

Next, detailed description follows regarding Examples and Comparative Examples of the technology disclosed herein. However, the technology disclosed herein is not limited to the content of the Examples described below.

The analysis device 12 of the first exemplary embodiment was employed in the Examples and Comparative Examples, and the concentration of endotoxins in sample water produced was analyzed by injecting endotoxins into carrier water so as to give an endotoxin concentration of 10 nM. Specifically, a measurement value range of the endotoxin concentration of the sample water was validated by the analyzer 48.

Measurement conditions in the Examples and Comparative Examples were an excitation wavelength of 350 nm, a detection florescent light wavelength of 500 nm, florescence reagent of Zn-dpa-C2OPy (see Chemical Equation (1) below), and the florescence reagent concentration in the reagent injection section was 10 µM.

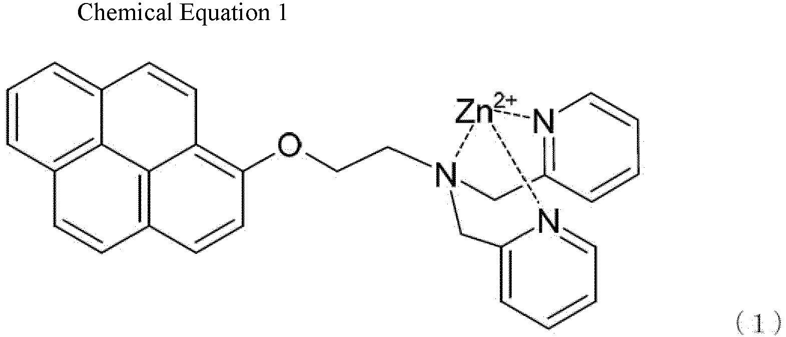

The measurement conditions in the Examples and Comparative Examples were an excitation wavelength of 350 nm and a detection florescent light wavelength of 500 nm.

Table 1 lists a pressure P1 at an inlet side of the membrane filtration device 16, a pressure P2 at the outlet port of the pump 42, and a measurement value range in the analyzer 48 for each of the Examples and Comparative Examples. The measurement value ranges are ranges of values actually measured as endotoxin concentrations in the analyzer 48, and specifically are the maximum values and the minimum values obtained for measurement that was performed 5 times.

The pressure P1 at the inlet side of the membrane filtration device 16 is able to achieve a desired pressure using the non-illustrated water treatment system disposed at the upstream side of the analysis device 12, and the front concentration pump 14.

The Examples 1 to 7 and the Comparative Examples 1 to 3 listed in Table 1 indicate validated measurement value ranges for changed values set for the pressure P1 and the pressure P2 in the analysis device 12 as illustrated in Fig. 1.

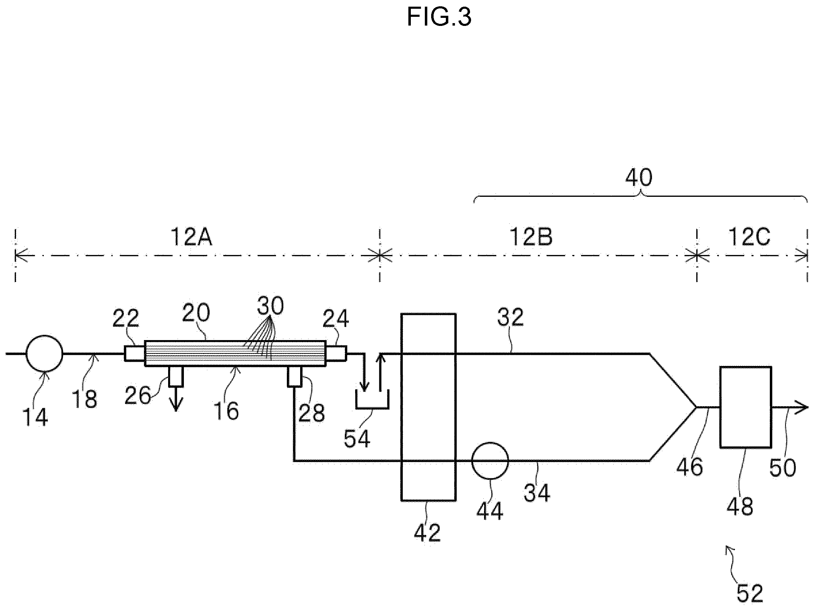

Moreover, Example 8 in Table 1 is an Example for a case in which the analysis device 52 illustrated in Fig. 3 was employed. In the analysis device 52, a small tank 54 is further installed between the outlet port 24 and the pump 42 of the analysis device 12 illustrated in Fig. 1. In such cases the sample water concentrated by the membrane filtration device 16 is temporarily stored in the tank 54, and this stored sample water is sucked in by the pump 42. Note that the volume of the tank 54 is 10 ml.

**Table 1**

| | P1 (MPa) | P2 (MPa) | Measurement Value Range (nM) |
|---|---|---|---|
| Example 1 | 0.06 | 0.50 | 9.3 to 10.3 |
| Example 2 | 0.12 | 0.50 | 9.5 to 10.3 |
| Example 3 | 0.18 | 0.25 | 9.9 to 10.1 |
| Example 4 | 0.18 | 0.50 | 9.8 to 10.1 |
| Example 5 | 0.18 | 0.75 | 9.8 to 10.2 |
| Example 6 | 0.18 | 0.99 | 9.7 to 10.3 |
| Example 7 | 0.03 | 0.50 | 8.5 to 11.5 |
| Example 8 | 0.06 | 0.50 | 9.9 to 14.0 |
| Comparative Example 1 | 0.06 | 0.06 | 0.8 to 2.0 |
| Comparative Example 2 | 0.12 | 0.11 | 0.5 to 1.2 |
| Comparative Example 3 | 0.18 | 0.17 | 0.1 to 0.9 |

As is apparent from Table 1, in Examples 1 to 7, which satisfy the relationship P1 < P2, substantially the same measurement values are obtained, as the measurement value range, to the endotoxin concentration of the supply water. Namely, Examples 1 to 7 enable quantitative measurement of the endotoxin concentration for the sample water. This is because in the Examples 1 to 7, the flow rate of sample water flowing in the first piping 32 is controlled appropriately and there is an appropriate value for a flow rate ratio between this sample water and the carrier water flowing in the second piping 34. The analyzer 48 functions correctly due to the endotoxin concentration of the concentrated water being high.

For Example 8, the pressures P1 and P2 are standard values and the relationship P1 < P2 is satisfied. In Example 8 there is a tendency for the numerical value of the measurement value range to be larger than in the other Examples. This is due to contamination occurring in the tank 54 when the sample water is received in the tank 54. Because it is known in advance for the Example 8 that the numerical values of the measurement value range as measured will be larger than actual values, specific correction coefficients corresponding thereto may, for example, be obtained and these correction coefficients used to multiply the measured numerical values.

Example 8 has an advantage in that by employing the tank 54, the pressure P1 can be freely set due to pressure at earlier stages to the tank 54 not affecting stages following the tank 54.

In particular, as the value of pressure P1, the condition P1 < P2 is satisfied, and preferably P1 ≥ 0.03 MPa, and more preferably P1 ≥ 0.05 MPa. By setting a lower limit value of the value of the pressure P1 in this manner, concentration can be performed sufficiently with the ultrafiltration membrane 30, and measurement accuracy is higher. Note that from out of the Examples illustrated in Table 1, the smallest value of pressure P1 is 0.03 MPa and the next smallest value is 0.06 MPa, and in practice concentration can be performed sufficiently with the ultrafiltration membrane 30 as long as the pressure P1 is P1 ≥ 0.05 MPa.

In contrast thereto, the relationship P1 ≥ P2 is satisfied in Comparative Examples 1 to 3, and a measurement value range is a smaller numerical value than the concentration of endotoxins in the supply water. This is because in the Comparative Examples 1 to 3 a large volume of sample water flows in the first piping 32, the flow rate ratio between the sample water and the carrier water flowing in the second piping 34 is not correct, and there is a dilute reagent concentration in the merge piping 46 or an unstable concentration therein. Moreover, due to the amount of sample water flowing in the first piping 32 not being stabilized and not being able to maintain a high endotoxin concentration, this results in a state in which the measurement values of the analyzer 48 do not reach an appropriate measurement value range.

Note that instead of the tank 54, installation of a pressure adjustment valve might be considered in the analysis device 52 illustrated in Fig. 3. However, such a method is not able to be implemented in practice due to there being no valve capable of adjusting the pressure at such levels of flow rates commercially available.

The following supplements are also disclosed.

### Supplement 1

An analysis device including:
a membrane filtration device provided with a microfiltration membrane or an ultrafiltration membrane;
a flow injection system that analyzes sample water from a reaction between a detection target contained in the sample water and a reagent that is contained in carrier water and that reacts with the detection target;
first piping that passes concentrated water, in which the detection target has been concentrated by the membrane filtration device, to the flow injection system as the sample water; and
a pump that pressurizes the sample water in the first piping.

### Supplement 2

The analysis device of Supplement 1, wherein an internal pipe diameter of an outlet pipe in the flow injection system is less than an internal pipe diameter of the first piping.

### Supplement 3

The analysis device of Supplement 1 or Supplement 2, wherein:
the membrane filtration device includes a discharge section that is open externally and that discharges pass-through water that has passed through the ultrafiltration membrane.

### Supplement 4

The analysis device of Supplement 3, further including second piping that passes at least part of pass-through water of the membrane filtration device to the flow injection system as the carrier water.

### Supplement 5

The analysis device of Supplement 4, wherein the membrane filtration device includes a connection port to which the second piping is connected.

### Supplement 6

The analysis device of Supplement 1, wherein a pressure at an inlet side of the membrane filtration device is less than a pressure at an outlet port of the pump and is 0.05 MPa or higher.

### Supplement 7

An analysis method including:
generating sample water in which a detection target has been concentrated by a membrane filtration device provided with a microfiltration membrane or an ultrafiltration membrane;
pressurizing the sample water with a pump and feeding the sample water to a flow injection system; and
analyzing the sample water in the flow injection system from a reaction between the detection target and a reagent that is contained in carrier water and that reacts with the detection target.

### Supplement 8

The analysis method of Supplement 7, wherein the water pressure at the downstream side of the pump is higher than the water pressure at the upstream side of the pump.

### Supplement 9

The analysis method of Supplement 7 or Supplement 8, wherein in the membrane filtration device, pass-through water that has passed through the ultrafiltration membrane is discharged externally from the membrane filtration device in an unpressurized state.

### Supplement 10

The analysis method of Supplement 9, wherein at least part of the pass-through water of the membrane filtration device is fed to the flow injection system as the carrier water. Supplement 11

The analysis method of Supplement 7, wherein a pressure at an inlet side of the membrane filtration device is less than a pressure at an outlet port of the pump and is 0.05 MPa or higher.

The entire content of the disclosure of Japanese Patent Application No. 2023-210477 filed on December 13, 2023 is incorporated by reference in the present specification.

All publications, patent applications and technical standards mentioned in the present specification are incorporated by reference in the present specification to the same extent as if each individual publication, patent application, or technical standard was specifically and individually indicated to be incorporated by reference.

## Claims

1. An analysis device, comprising:
a membrane filtration device provided with a microfiltration membrane or an ultrafiltration membrane;
a flow injection system that analyzes sample water from a reaction between a detection target contained in the sample water and a reagent that is contained in carrier water and that reacts with the detection target;
first piping that passes concentrated water, in which the detection target has been concentrated by the membrane filtration device, to the flow injection system as the sample water; and
a sample water pump that pressurizes the sample water in the first piping,
wherein an internal pipe diameter of an outlet pipe in the flow injection system is smaller than an internal pipe diameter of the first piping.

2. An analysis device, comprising:
a membrane filtration device provided with a microfiltration membrane or an ultrafiltration membrane;
a flow injection system that analyzes sample water from a reaction between a detection target contained in the sample water and a reagent that is contained in carrier water and that reacts with the detection target;
first piping that passes concentrated water, in which the detection target has been concentrated by the membrane filtration device, to the flow injection system as the sample water;
second piping that passes at least part of pass-through water of the membrane filtration device to the flow injection system as the carrier water; and
a sample water pump that pressurizes the sample water in the first piping.

3. An analysis device, comprising:
a membrane filtration device provided with a microfiltration membrane or an ultrafiltration membrane;
a flow injection system that analyzes sample water from a reaction between a detection target contained in the sample water and a reagent that is contained in carrier water and that reacts with the detection target;
first piping that passes concentrated water, in which the detection target has been concentrated by the membrane filtration device, to the flow injection system as the sample water;
second piping that passes the carrier water to the flow injection system; and
a sample water pump that pressurizes the sample water in the first piping,
wherein the membrane filtration device includes a connection port to which the second piping is connected.

4. An analysis device, comprising:
a membrane filtration device provided with a microfiltration membrane or an ultrafiltration membrane;
a flow injection system that analyzes sample water from a reaction between a detection target contained in the sample water and a reagent that is contained in carrier water and that reacts with the detection target;
first piping that passes concentrated water, in which the detection target has been concentrated by the membrane filtration device, to the flow injection system as the sample water; and
a sample water pump that pressurizes the sample water in the first piping,
wherein a pressure at an inlet side of the membrane filtration device is less than a pressure at an outlet port of the sample water pump and is 0.05 MPa or higher.

5. The analysis device of claim 2 or claim 3, wherein:
the membrane filtration device includes a discharge section that is open externally and that discharges pass-through water that has passed through the microfiltration membrane or the ultrafiltration membrane; and
a linear velocity of the pass-through water discharged from the discharge section is from 0.1 m/s to 2.0 m/s.

6. The analysis device of claim 1 or claim 4, wherein:
the membrane filtration device includes a discharge section that is open externally and that discharges pass-through water that has passed through the microfiltration membrane or the ultrafiltration membrane;
a linear velocity of the pass-through water discharged from the discharge section is from 0.1 m/s to 2.0 m/s; and
the analysis device further comprises second piping that passes at least part of the pass-through water to the flow injection system as the carrier water.

7. An analysis method, comprising:
generating sample water in which a detection target has been concentrated by a membrane filtration device provided with a microfiltration membrane or an ultrafiltration membrane;
pressurizing the sample water with a sample water pump and feeding the sample water to a flow injection system;
analyzing the sample water in the flow injection system from a reaction between the detection target and a reagent that is contained in carrier water and that reacts with the detection target; and
setting a water pressure at a downstream side of the sample water pump higher than a water pressure at an upstream side of the sample water pump.

8. An analysis method, comprising:
generating sample water in which a detection target has been concentrated by a membrane filtration device provided with a microfiltration membrane or an ultrafiltration membrane;
pressurizing the sample water with a sample water pump and feeding the sample water to a flow injection system;
feeding at least part of pass-through water that has passed through the microfiltration membrane or the ultrafiltration membrane in the membrane filtration device as carrier water to the flow injection system; and
analyzing the sample water in the flow injection system from a reaction between the detection target and a reagent that is contained in the carrier water and that reacts with the detection target.

9. An analysis method, comprising:
generating sample water in which a detection target has been concentrated by a membrane filtration device provided with a microfiltration membrane or an ultrafiltration membrane;
pressurizing the sample water with a sample water pump and feeding the sample water to a flow injection system;
setting a pressure at an inlet side of the membrane filtration device to less than a pressure at an outlet port of the sample water pump and to 0.05 MPa or higher; and
analyzing the sample water in the flow injection system from a reaction between the detection target and a reagent that is contained in carrier water and that reacts with the detection target.

10. The analysis method of claim 8, wherein, in the membrane filtration device, pass-through water that has passed through the microfiltration membrane or the ultrafiltration membrane is externally discharged in an unpressurized state from a discharge section of the membrane filtration device and at a linear velocity of from 0.1 m/s to 2.0 m/s.

11. The analysis method of claim 7 or claim 9, wherein, in the membrane filtration device:
pass-through water that has passed through the microfiltration membrane or the ultrafiltration membrane is externally discharged in an unpressurized state from a discharge section of the membrane filtration device and at a linear velocity of from 0.1 m/s to 2.0 m/s; and
at least part of the pass-through water that has passed through the microfiltration membrane or the ultrafiltration membrane of the membrane filtration device is fed as carrier water to the flow injection system.
